# EUROPÄISCHE PATENTSCHRIFT

(11) **EP 1 709 205 B1**
(45) Veröffentlichungstag und Bekanntmachung des Hinweises auf die Patenterteilung: **13.05.2009**
(21) Anmeldenummer: 05730703.5
(22) Anmeldetag: 28.01.2005
(51) Int. Cl.: C12Q 1/68

(54) **DIAGNOSE VON UNIPARENTALER DISOMIE ANHAND VON SINGEL-NUKLEOTID-POLYMORPHISMEN**
DIAGNOSIS OF UNIPARENTAL DISOMY WITH THE AID OF SINGLE NUCLEOTIDE POLYMORPHISMS
DIAGNOSTIC DE DISOMIE UNIPARENTALE A L'AIDE DE POLYMORPHISMES D'UN SEUL NUCLEOTIDE

(30) Priorität: 30.01.2004 DE 102004005497
(43) Veröffentlichungstag der Anmeldung: 11.10.2006
(73) Patentinhaber: Eberhard-Karls-Universität Tübingen, 72076 Tübingen (DE)
(72) Erfinder: BONIN, Michael, 72108 Rottenburg (DE); ALTUG-TEBER, Oezge, 71696 Möglingen (DE); RIESS, Olaf, 72070 Tübingen (DE)
(74) Vertreter: Findeisen, Marco
(86) Internationale Anmeldenummer: PCT/EP2005/000855
(87) Internationale Veröffentlichungsnummer: WO 2005/090598

(56) Entgegenhaltungen:
- WO-A-99/01580
- ROBINSON W.P.: "Mechanisms leading to uniparental disomy and their clinical consequences" BIOESSAYS, Bd. 22, Nr. 5, Mai 2000 (2000-05), Seiten 452-459, XP002328255 ISSN: 0265-9247 in der Anmeldung erwähnt
- LALANDE M.: "Parental imprinting and human disease" ANNUAL REVIEW OF GENETICS 1996 UNITED STATES, Bd. 30, 1996, Seiten 173-195, XP002328257 ISSN: 0066-4197
- EGGERMANN T. ET AL.: "Uniparental disomy: Clinical indications for testing in growth retardation" EUROPEAN JOURNAL OF PEDIATRICS, Bd. 161, Nr. 6, Juni 2002 (2002-06), Seiten 305-312, XP002328256 ISSN: 0340-6199 in der Anmeldung erwähnt
- EGGERMANN T. ET AL.: "Silver-Russell Syndrome (SRS): Present state of research and indications for an investigation uniparental disomy 7 (UPD7)" MEDIZINISCHE GENETIK GERMANY, Bd. 12, Nr. 3, 2000, Seiten 348-352, XP009047785 ISSN: 0936-5931
- CHOU Y.-Y. ET AL.: "Russell-Silver syndrome: molecular diagnosis of maternal uniparental disomy of chromosome 7 using methylation-specific polymerase chain reaction assay and single nucleotide polymorphisms genotyping." JOURNAL OF THE FORMOSAN MEDICAL ASSOCIATION = TAIWAN YI ZHI. OCT 2004, Bd. 103, Nr. 10, Oktober 2004 (2004-10), Seiten 797-802, XP009047775 ISSN: 0929-6646

## Beschreibung

Die vorliegende Erfindung betrifft ein Verfahren zur Diagnose von uniparentaler Disomie (UPD) bei einem menschlichen Lebewesen und die Verwendung von in einer biologischen Probe eines menschlichen Lebewesens vorliegenden Singel-Nukleotid-Polymorphismen (SNPs), die bei den beiden Elternteilen diametral entgegengesetzt homozygot vorligen (informative SNPs) zur Diagnose von UPD.

Verfahren der vorstehenden Art sind im Stand der Technik allgemein bekannt.

Unter uniparentaler Disomie (UPD) wird das genetische Phänomen verstanden, bei dem bei einem Lebewesen durch das Ausbleiben der Trennung eines Chromosomenpaares oder Fragmenten des Chromosomenpaars (engl.: "non-disjunction") in der Meiose oder Mitose entgegen der natürlichen Situation beide homologen Chromosomen oder entsprechende Chromosomenfragmente von nur einem Elternteil vorliegen. Bei einer UPD findet man entweder die beiden homologen Chromosomen (Heterodisomie) oder zwei Kopien eines homologen Chromosoms (Isodisomie) oder eine Mischung von heterodisomen und isodiomen Segmenten. Je nachdem, welches Allel ausfällt, spricht man beim Ausfall des väterlichen Allels von maternaler, beim Ausfall des mütterlichen Allels von paternaler Disomie.

Klinische Konsequenzen einer UPD ergeben sich bei einer Homozygotie autosomal rezessiver Erbanlagen, durch Mosaiktrisomien oder durch die Veränderung der Expression von Genen, die der genomischen Prägung (engl.: "genomic imprinting") unterliegen. Bei dem epigenetischen Prozess der Prägung werden bestimmte Chromosomenabschnitte durch die männliche und weibliche Keimbahn spezifisch markiert, so dass in somatischen Zellen entweder nur das väterliche oder das mütterliche Allel eines Gens aktiv ist. Uniparentale Disomien können hier zum vollständigen Funktionsverlust geprägter Gene in den entsprechenden Chromosomenabschnitten und dadurch zu bestimmten Krankheitsbildern führen.

Uniparentale Disomien werden häufig bei Fehlgeburten beobachtet. In 50 % aller Fälle findet man numerische Chromosomenaberrationen. Die Häufigkeit einer UPD, so wird vermutet, liegt bei diesen Zygoten bei etwa 3:10.000; vgl. Engel, E. (1980) A new genetic concept: uniparental disomy and its potential effect, isodisomie, Am J Med Genet. 6(2): 137-143. Das Risiko von numerischen Chromosomenaberrationen und damit von UPD steigt mit dem Alter der Mütter. Allein eine uniparentale Disomie betreffend das Chromosom 15 (UPD 15) wird bei 1:2.40 0 lebend Geborenen der Mütter von über 40 Jahren abgeschätzt; vgl. Lalande, M. (1997), "Parental imprinting and human disease", Rev. Genet. 30: 173-195.

Die möglichen klinischen Konsequenzen einer UPD sind in nachfolgender Tabelle 1 zusammengefasst (nach Robinson, W. P. (2000), "Mechanisms leading to uniparental disomy and their clinical consequences", Bioessays 22(5): 452-459).

### Tabelle 1: Mögliche klinische Konsequenzen einer UPD

- Homozygotie für eine rezessive Mutation,
- Störung des Imprinting-Prozesses,
- intrauterine Wachstumsretardierung mit postnataler Entwicklungsverzögerung und fetale Malformationen wegen der Zellen mit einer UPD im Mutterkuchen und/oder fetalen Gewebe,
- Fertilitätsprobleme, Krebs und andere Erkrankungen (falls die anormalen Zellen nach der Geburt persistieren),
- ein milder Phänotyp bei Mosaiken (Vorhandensein von Zellen mit und ohne UPD),
- Altersbedingte komplexe Eigenschaften wie z.B. bei Krebserkrankungen aufgrund LOH (Verlust der Heterozygotie, englisch: "loss of heterozygosity") oder LOI (Verlust der Prägung, englisch: "loss of imprinting") an der entsprechenden Stelle eines Chromosoms.

Eine UPD geht, wie der vorstehenden Tabelle 1 zu entnehmen ist, häufig einher mit einer IUGR bzw. PGR (engl.: "intrauterine growth retardation", "postnatal growth retardation"). Insbesondere liegen IUGR und PGR bei den UPDs von Chromosom 16 und 20 als ausgeprägte klinische Zeichen vor; vgl. Eggermann, E. et al. (2002), Uniparental disomy, clinical indications for testing in growth retardation, Eur J Pediatr. 161(6): 305-12.

Die klassischen Beispiele für eine UPD mit charakteristischem Phänotyp sind das Prader-Willi-Syndrom, das durch eine maternale uniparentale Disomie des Chromosoms 15 gekennzeichnet ist (PWS; matUPD15), das Angelmann-Syndrom (AS; patUPD15) und das Silver-Russell-Syndrom (SRS; matUPD7). Außerdem wurden klinische Auffälligkeiten bei maternalen uniparentalen Disomien für die Chromosomen 14 (Kleinwuchs mit Entwicklungsretardierung), 6 (transienter neonataler Diabetis mellitus), 11 (Beckwith-Wiedemann-Syndrom) und 14 (starke Ausprägung von Kleinwuchs mit mentaler Retardierung) beschrieben. Es wird vermutet, dass auch uniparentale Disomien anderer Chromosomen einen Einfluss auf Wachstum und Entwicklung haben.

Man kennt eine Vielzahl von Krankheiten, die aufgrund einer UPD entstehen können und in nachfolgender Tabelle 2 zusammengefasst sind (nach Engel, E. und Antonorakis, S. E. (2002), Genomic imprinting and uniparental disomy in medicine, Clinical and Molecular Aspects, Wiley-Liss).

**Tabelle 2:**

| Autosomal-rezessiv erbliche Erkrankungen infolge einer UPD | | |
|---|---|---|
| Erkrankung | Gen | UPD |
| Junctional epidermolysis bullosa | LAMB3 | matUPD1 |
| Junctional epidermolysis bullosa | LAMC2 | patUPD1 |
| Chediak-Higashi-Syndrom | LYST | matUPD1 |
| Pycnodysostosis | CTSK | patUPD1 |
| Congenital pain insensitivity with anhydrosis (CIPA) | TRKA | patUPD1 |
| 5-Alpha-Reduktase 2-Mangel | SRD5A2 | patUPD2 |
| Abetalipoproteinämie | MTP | matUPD4 |
| Spinale Muskelatrophie | SMA | patUPD5 |
| Komplement-C4-Mangel | C4 | patUPD6 |
| Methylmalonicacidämie | MUT | patUPD6 |
| 21-Hydroxylasemangel | CYP21 | patUPD6 |
| Cystische Fibrose | CFTR | matUPD7 |
| Osteogenesis Imperfecta | COL1A2 | matUPD7 |
| Kongenitale Chlorid-diarrhea | DRA | matUPD7 |
| Cystische Fibrose und Immotil-Cilia-Syndrom | CFTR | matUPD7 |
| Lipoproteinlipasemangel | LPL | patUPD8 |
| Knorpel-Haar-Hypoplasie | RMRP | matUPD9 |
| Leigh-Syndrom | SURF1 | matUPD9 |
| Beta-Thalassämie | HBB | patUPD11 |
| Rod Monochromacy | | matUPD14 |
| Bloom-Syndrom | BLM | matUPD15 |
| Alpha-Thalassämie | HBA | patUPD16 |

Im Stand der Technik erfolgt die Diagnose einer UPD üblicherweise anhand von Mikrosatelliten als genetische Marker. Bei Mikrosatelliten handelt es sich um tandemartige, sich wiederholende kurze DNA-Motive, bestehend aus Nukleotidmotiven von bis zu sechs Basenpaaren. Diese kommen im menschlichen Genom verteilt vor. Mikrosatelliten sind hoch polymorph, d.h. die Mikrosatelliten von zwei nicht verwandten Individuen unterscheiden sich mit hoher Wahrscheinlichkeit.

Mikrosatelliten vererben sich gemäß der Mendel'schen Regeln. Ob eine UPD vorliegt, wird bislang über die Analyse der Vererbung von Mikrosatelliten untersucht. Hierfür wird deshalb die DNA der zu untersuchenden Person und der Eltern benötigt. Diese DNA wird in mehreren Polymerase-Kettenreaktionen (PCR) auf mehrere Mikrosatelliten-Marker hin untersucht. Da sich die Mikrosatelliten des mütterlichen Elternteils von denen des väterlichen Elternteils unterscheiden, kann festgestellt werden, ob bspw. bezüglich eines bestimmten Chromosoms ausschließlich väterliche Mikrosatelliten vererbt wurden und deshalb eine paternale UPD vorliegt.

Diese bekannte UPD-Diagnostik, bei der die Vererbung von Mikrosatelliten analysiert wird, birgt eine Vielzahl von Nachteilen: Für eine zuverlässige UPD-Diagnose benötigt man Kenntnis über eine beachtliche Anzahl von Mikrosatelliten, d.h. für die zu untersuchenden Abschnitte im Genom müssen Mikrosatelliten sowie zusätzlich deren flankierende Sequenzen bekannt sein. Das bedeutet, viele ggf. bislang unbekannte UPDs bleiben häufig aufgrund der fehlenden Kenntnis entsprechend lokalisierter Mikrosatelliten unentdeckt. Ferner ist diese UPD-Diagnostik sehr zeit- und arbeitsaufwändig (die Diagnose eines Prader-Willi-Syndroms bzw. Angelmann-Syndroms benötigt zwei bis vier Wochen) und wird deshalb nur bei hinreichendem Verdacht auf eine UPD durchgeführt.

Ein Überblick über das im Stand der Technik durchgeführte UPD-Diagnoseverfahren wird bei Horsthemke, B. et al. (2001), "Leitlinien für die molekulare und zytogenetische Diagnostik bei Prader-Willi-Syndrom und Angelmann-Syndrom", Verlag Medizinische Genetik, Sonderdruck, 7. Auflage, 78-80, gegeben.

Aufgabe der vorliegenden Erfindung ist es deshalb, ein Verfahren zur Diagnose von UPD bei einem menschlichen Lebewesen bereitzustellen, mit dem vorstehend beschriebene Nachteile vermieden werden. Insbesondere soll ein schnell und mittels geringem Personal- und Kostenaufwand durchführbares Verfahren bereitgestellt werden, das zuverlässige Ergebnisse liefert und mittels dem auch die Erfassung von bisher unbekannten UPDs möglich ist.

Diese Aufgabe wird durch ein Verfahren zur Diagnose von UPD bei einem menschlichen Lebewesen gelöst, das folgende Schritte aufweist: (1) Bereitstellung einer biologischen Probe des menschlichen Lebewesens sowie von biologischen Proben beider Elternteile des menschlichen Lebewesens; (2) Genotypisierung von Singel-Nukleotid-Polymorphismen (SNPs) des Lebewesens und der beiden Elternteile anhand der biologischen Proben; (3) Auswahl von solchen SNPs, die bei den beiden Elternteilen diametral entgegengesetzt homozygot vorliegen (informative SNPs); (4) Vergleich der Genotypen der informativen SNPs mit dem Genotyp der entsprechenden SNPs des menschlichen Lebewesens, und (5) Korrelation einer Homozygotie der SNPs des menschlichen Lebewesens aus Schritt (4) mit dem Vorliegen einer UPD.

Mittels dieses Verfahrens wird die der Erfindung zugrunde liegende Aufgabe vollständig gelöst.

Im Rahmen der Erfindung wird unter einer biologischen Probe eine solche verstanden, die genomische DNA des zu untersuchenden menschlichen Lebewesens enthält, wie beispielsweise eine Blutprobe, Speichelprobe, ein Abstrich, eine sonstige zellenenthaltende Probe, etc.

Unter SNP wird das genetische Phänomen verstanden, nach dem sich die Sequenzen zweier nicht verwandter Personen an etwa jeder 1000sten Stelle in einem einzelnen Basenpaar unterscheiden. Mit anderen Worten, ein SNP bezeichnet eine Position im Genom, an der alternativ zwei verschiedene Basen mit einer Häufigkeit von mehr als 1 % auftauchen. SNPs sind biallelisch, d.h. sie kommen nur in zwei Alternativen (kurz A oder B) vor. Die Ausprägung für einen SNP in einem menschlichen Lebewesen kann drei verschiedene Genotypen annehmen, nämlich homozygot A (AA), homozygot B (BB) oder heterozygot AB (AB).

In dem Verfahren wird nach dem Vergleich des Genotyps der entsprechenden SNPs der beiden Eltern die Vererbung dieser SNPs im Kind, d.h. im zu untersuchenden menschlichen Lebewesen, überprüft. Dabei werden erfindungsgemäß bei beiden Elternteilen informative SNPs ausgewählt. Darunter versteht man SNPs, die bei den Eltern diametral entgegengesetzt homozygot vorliegen, bspw. weist der Vater einen Genotyp AA und die Mutter einen Genotyp BB der entsprechenden SNPs auf. Ein nicht von einer UPD betroffenes Kind zeigt dann stets eine heterozygote Ausprägung (AB des entsprechenden SNP). Findet man hingegen bei dem entsprechenden SNP des Kindes bzw. des zu untersuchenden Lebewesens eine Homozygotie, unabhängig welcher Ausprägung (AA oder BB), so lässt dies Rückschlüsse auf das Vorliegen einer uniparentalen Disomie in diesem genomischen Bereich zu. Bei diesem Beispiel entspräche die Homozygotie AA des Kindes einer paternalen UPD und die Homozygotie BB einer maternalen UPD.

Im Gegensatz zu Mikrosatelliten kennt man eine extrem hohe Zahl von SNPs, die mit einem durchschnittlichen Abstand von 1000 Bp über das gesamte menschliche Genom verteilt und in Datenbanken (http://www.ncbi.nlm.nih.gov/SNP/index.html) abrufbar sind. Bisher wurden fast 1,8 Mio. SNPs im menschlichen Genom aufgefunden (vgl. http://snp.cshl.org/). Aufgrund dieser großen Anzahl von bekannten SNPs und deren Verteilung über das gesamte Genom hinweg können über die Analyse der Vererbung von informativen SNPs auch bislang noch unbekannte Formen der UPD erfasst werden, d.h. auch betreffend Bereiche des Genoms, für die keine Mikrosatelliten bekannt sind. Insbesondere ist dazu auch nicht die Kenntnis der diese SNPs flankierenden Sequenzen erforderlich.

SNPs sind aufgrund ihrer biallelischen Form deutlich informationsärmer als Mikrosatelliten, die aufgrund ihrer Länge von bis zu sechs Basenpaaren mehrere tausend genotypische Ausprägungen aufweisen können. Deshalb wurde im Stand der Technik bislang angenommen, dass SNPs als genetische Marker für den Nachweis einer UPD nicht geeignet seien.

Zur Genotypisierung der SNPs können erfindungsgemäß sämtliche Methoden eingesetzt werden, mittels derer vorzugsweise über größere Bereiche des Genoms hinweg SNPs analysiert werden können. Beispiele hierfür sind der GeneChip®-Mapping 10K-Array der Firma Affymetrix®, mit dem 11.555 SNPs parallel gemessen werden können sowie weitere Arrays der Firma Affymetrix® (vgl.: www.affymetrix.com), oder die BeadArray-Technologie der Firma Illumina, mit der Möglichkeit, 4600 SNPs parallel zu messen (vgl.: www.illumina.com).

Im Vergleich zur Analyse der Vererbung von Mikrosatelliten ist das erfindungsgemäße Verfahren schnell, mit geringem Personalaufwand und verhältnismäßig niedrigen Kosten durchführbar. Hinzu kommt, dass für eine zuverlässige Diagnose von UPD geringe Mengen von DNA des zu untersuchenden Lebewesens, nämlich im Nanogrammbereich, ausreichend sind.

Wie die Erfinder zeigen konnten, kann auf Grund der Genauigkeit des neuen Verfahrens eine Fehldiagnose weitgehend vermieden werden.

Erfindungsgemäß ist bevorzugt, wenn in Schritt (2) des Verfahrens die Genotypisierung von SNPs von einem Chromosom erfolgt.

Mittels dieser Maßnahme wird sichergestellt, dass eine ausreichende Anzahl von SNPs analysiert wird, so dass eine zuverlässige Diagnose von UPD ermöglicht wird. Die Gefahr einer fehlerhaften Diagnose wird dadurch reduziert.

Erfindungsgemäß ist es ferner bevorzugt, wenn in Schritt (2) des Verfahrens eine genomweite Genotypisierung von SNPs erfolgt. Diese kann mittels des GeneChip®-Mapping 10K-Arrays, 100K-Arrays oder 10K-Arrays 2.0 von Affymetrix® erfolgen.

Sämtliche Verfahren haben sich als besonders geeignet für deren Einsatz im Rahmen des erfindungsgemäßen Verfahrens erwiesen. So können mit dem GeneChip®-Mapping 100K-Array, das aus einem Set von zwei Arrays besteht, mehr als 100.000 SNPs mit einem einzigen Primer genotypisiert werden. Der GeneChip®-Mapping 10K-Array 2.0 verwendet Fotolithographietechnologie, wodurch ein kleineres und kosteneffizienteres Format ermöglicht wird.

Wie erwähnt, sind bei der im Stand der Technik bekannten Analyse der Vererbung von Mikrosatelliten Sequenzinformationen über die zu untersuchenden Bereiche erforderlich. Dies ist bei dem erfindungsgemäßen Verfahren nicht der Fall, so dass gemäß dieser Ausführungsform noch bislang unbekannte uniparentale Disomien genomweit und auch in Bereichen auffindbar sind, in denen keine Mikrosatelliten bekannt sind. So sind die Ursachen von Minderwuchs bzw. Großwuchssymptomatik und Entwicklungsverzögerung bei Kindern bisher häufig unbekannt, und es wird vermutet, dass eine Vielzahl der betroffenen Kinder eine ursächliche UPD haben. Die vorliegende Erfindung bietet nun erstmals die Möglichkeit, bei diesen Kindern eine genomweite Genotypisierung der SNPs durchzuführen und diagnostisch abzuklären, ob in der Tat eine UPD vorliegt oder nicht.

Bei dieser erfindungsgemäßen Ausführungsform werden DNA-Mengen von lediglich ca. 250 Nanogramm benötigt, wohingegen bei Anwendung der bisherigen Verfahren bei einer genomweiten Analyse auf UPD sehr große Mengen im Mikrogrammbereich erforderlich wären und eine entsprechende Untersuchung ferner mehrere Monate in Anspruch nehmen würde. Hierzu im Gegensatz gelingt mittels der Erfindung die Diagnose von UPD auf der Basis einer genomweiten Genotypisierung von SNPs innerhalb von drei Tagen. Das erfindungsgemäße Verfahren ist deshalb auch als Routineuntersuchung geeignet.

Durch die hochdichte Verteilung von SNPs - der durchschnittliche Abstand zwischen zwei SNPs liegt bei 210 kB - ist gerade die Detektierbarkeit von kleinen segmentalen UPDs im Vergleich zur Mikrosatellitenanalyse - durchschnittlicher Abstand von Mikrosatelliten im Standardset (etwa 400 Mikrosatelliten; vgl. bspw. www.licor.com) beträgt 5,6 MB (10 cM) - verbessert möglich.

Die Verwendung des GeneChip®-Mapping 10K-Arrays von Affymetrix® hat den Vorteil, dass dieses einfach handhabbar ist, da es als Mikroarray konzipiert ist, über 10.000 SNPs genomweit analysiert werden können, sehr geringe Ausgangs-DNA-Mengen benötigt werden und eine sehr hohe Sensitivität und damit Zuverlässigkeit gewährleistet ist. Nähere Ausführungen zu dieser Methode können dem Handbuch entnommen werden (Manual, Ausgabe 2003).

Dabei ist es bevorzugt, wenn das erfindungsgemäße Verfahren bei einem menschlichen Lebewesen durchgeführt wird, das Anzeichen einer intrauterinen Wachstumshemmung (IUGR) mit einer postnatalen Entwicklungsverzögerung (PGR) zeigt.

Intrauterine Wachstumsretardierung (IUGR) mit postnataler Entwicklungsverzögerung (PGR) gehen bekanntermaßen häufig, jedoch nicht immer, mit einer UPD einher, wobei eine Diagnose wegen des hohen Kosten- und Personalaufwandes selbst bei betroffenen Patienten häufig vermieden wurde. Bei diesen Personen kann nun mittels des erfindungsgemäßen Verfahrens erstmals auch routinemäßig und mit geringem Aufwand eine sichere Diagnose vorgenommen werden, ob in der Tat eine UPD vorliegt oder nicht, und damit die Basis für eine anschließende zielgerichtete Therapie geschaffen werden.

In diesem Zusammenhang ist es besonders bevorzugt, wenn das Verfahren bei einem menschlichen Lebewesen durchgeführt wird, das Anzeichen eines Prader-Willi-(PWS), Angelmann- (AS), Silver-Russell- (SRS) oder Beckwith-Wiedemann-Syndroms oder Anzeichen transienter neonataler Diabetes mellitus zeigt.

Diese Maßnahme hat den Vorteil, dass nun auch bei Anzeichen des Vorliegens dieser klassischerweise mit einer UPD assoziierten Syndrome bzw. Krankheitsbilder festgestellt werden kann, ob tatsächlich eine entsprechende genetische Situation vorliegt. Eine diagnostische Untersuchung betroffener Personen ist bislang nur mittels Mikrosatellitenanalyse möglich, wobei von dieser bislang wegen des großen Aufwandes häufig abgesehen wurde.

Vor diesem Hintergrund ist ein weiterer Gegenstand der vorliegenden Erfindung auch die Verwendung von in einer biologischen Probe eines menschlichen Lebewesens vorliegenden SNPs, die bei beiden Elternteilen diametral entgegengesetzt homozygot vorliegen (informative SNPs) zur Diagnose von UPD, insbesondere im Zusammenhang mit Verdacht auf IUGR/PGR, PWS, AS, SRS, Beckwith-Wiedemann-Syndrom oder transiente neonatale Diabetes mellitus.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Die vorliegende Erfindung wird nun anhand eines Ausführungsbeispiels näher erläutert, aus dem sich weitere Eigenschaften und Vorteile der vorliegenden Erfindung ergeben. Darin wird auf die beiliegenden Figuren Bezug genommen, in denen Folgendes dargestellt ist:

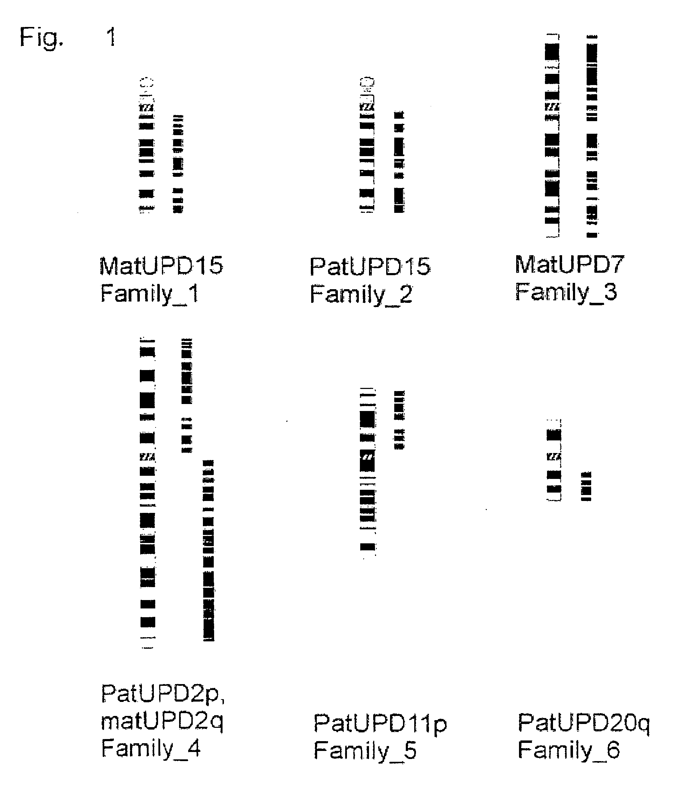

- Fig. 1: zeigt die schematische Darstellung von biallelischen SNPs mit chromosomalen Lokalisierungen in sechs nicht miteinander in Bezug stehenden Familien (Family_1 - Family_6). Jeder informative SNP (Marker) wird durch einen Balken auf der rechten Seite des Ideogramms dargestellt. Die Diagnose für jeden Fall ist unter den Ideogrammen angegeben (mat: maternal, pat: paternal). Die informativen Marker sind in drei Fällen mit vollständiger UPD über das gesamte Chromosom (oben, Familien 1 bis 3) und in drei Fällen mit segmentaler UPD (unten, Familien 4 bis 6) über eine chromosomale Region verteilt. In sämtlichen Fällen mit Ausnahme des maternalen UPD-15-Falles (Familie 2), zeigten die SNPs, die zwischen den informativen Markern liegen, eine Reduktion zur Homozygotie, wodurch eine Isodisomie angezeigt wird.

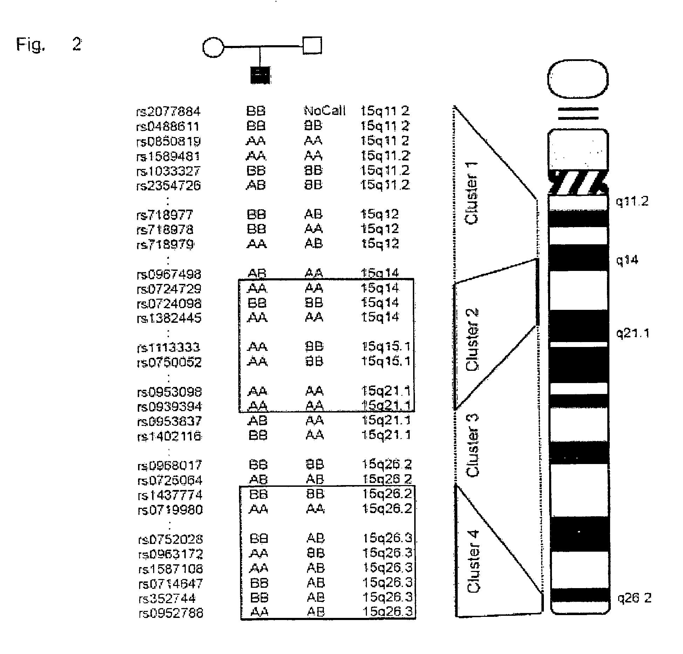

- Fig. 2: zeigt die Haplotyp-Analyse im Falle der maternalen UPD 15 entlang des Chromosoms 15 (Familie 2). Die Referenz-SNP-Identitätsnummer und die jeweiligen Genotypen der ausgewählten Marker sind unterhalb des Stammbaumes dargestellt (AA: Homozygot für ein Allel, BB: Homozygot für das andere Allel, AB: Heterozygot). Die chromosomalen Lokalisierungen dieser SNPs sind in der ganz rechts dargestellten Säule gelistet. Die Marker bei 15q14 bis q21.21 und bei 15q26.2 bis qter zeigten eine Reduktion zur Homozygotie, wodurch segmentale Isodisomie angezeigt wird (in den quadratischen Kästen). Die informativen Marker zwischen den heterozygoten SNPs entlang des gesamten Chromosoms 15 zeigen einen Fehler in der Meiose an.

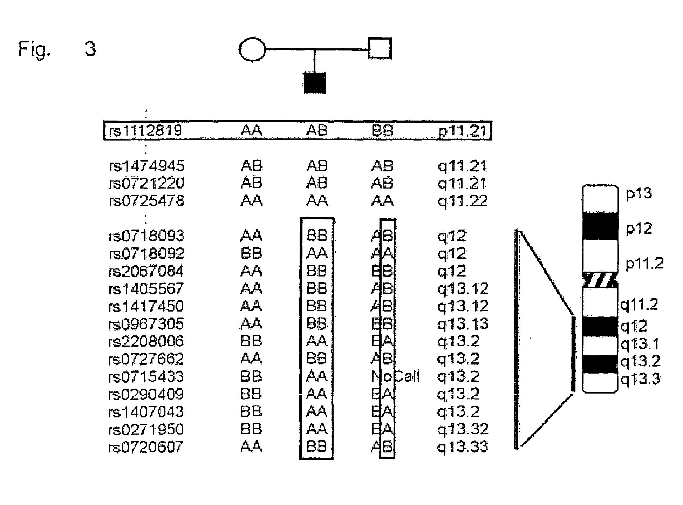

- Fig. 3: zeigt eine Haplotyp-Analyse des paternalen UPD20q-Falls mit einer Gruppe von Markern (Familie 6). Die Allele bei 20q12 bis qter (in den quadratischen Kästen) zeigen eine paternale Isodisomie mit dem Ausbleiben der maternalen Verteilung in die betroffenen Nachkommen an. Die SNPs in nächster Nähe zu dem Centromer waren nicht informativ und die bei 20p zeigten normale Mendel'sche Vererbung.

### Ausführungsbeispiel

### Untersuchte Patienten

Das erfindungsgemäße Verfahren wurde an sechs Patienten erprobt, die zuvor mittels des herkömmlichen Diagnoseverfahrens auf der Basis der Mikrosatellitenanalyse als vom Angelmann-Syndrom (Kind aus Familie 1; patUPD15), vom Prader-Willi-Syndrom (Kind aus Familie 2; matUPD15), als von Silver-Russell-Syndrom (Kind aus Familie 3; matUPD7), von habituellen Aborten, d.h. wiederholten Fehlgeburten (Kind aus Familie 4; patUPD2p), vom Beckwith-Wiedemann-Syndrom (Kind aus Familie 5; patUPD11p) bzw. als von Pseudo-Hypothyriodismus (Kind aus Familie 6; patUPD20) betroffen diagnostiziert wurden. Ferner wurden zu den jeweiligen Patienten bei vollständiger UPD ein Elternteil (Familien 1, 2 und 3), und bei segmentaler UPD (Familien 4, 5 und 6) beide Elternteile untersucht.

### Genotypisierung der SNPs der Elternteile und Patienten, Auswahl der informativen SNPs

Unter Verwendung des GeneChip®-Mapping 10K-Arrays von Affymatrix® (Affymetrix, Inc., Santa Clara, Vereinigte Staaten von Amerika) wurden die jeweiligen Elternteile und die Patienten gemäß der Anleitung (Manual) des Herstellers hinsichtlich der Genotypen der SNPs genomweit analysiert. Dazu wurden 250 ng der gesamten genomischen DNA mit einem Restriktionsenzym (XbaI) verdaut und an Adaptoren ligiert, die die kohäsiven vier Basenpaar langen Überhänge erkennen. Ein generischer Primer, der die Adaptersequenz erkennt, wurde verwendet, um in einem einzigen PCR-Schritt die an den Adapter ligierten DNA-Fragmente zu amplifizieren. Die amplifizierten und gereinigten PCR-Produkte wurden dann fragmentiert und mit Biotin-ddNTP markiert. Anschließend wurden die DNA-Fragmente einer jeden Probe in einem Affymetrix GeneChip-Hybridisierungsofen bei 48°C zu einem Single 10K-Array hybridisiert. Nach 16 Stunden wurden die Arrays gewaschen und auf der Affymetrix Fluidics Station 400 gefärbt. Für die Familien 1, 2 und 3 wurden die Arrays mit dem GeneArray Scanner 2500 und für die Familien 4, 5 und 6 mit dem GeneChip Scanner 3000 (Affymetrix, Inc.) gescannt.

Anschließend wurden die informativen SNPs ausgewählt, d.h. die SNPs, die bei den Elternteilen entgegengesetzt homozygot vorliegen. Basierend auf statistischen Überlegungen sollte bei einer genomweiten Analyse die Anzahl der informativen SNPs bei 25 bis 100 pro Familie liegen. Im Anschluss wurden die den informativen SNPs der Eltern entsprechenden SNPs ausgewählt und deren Ausprägung festgestellt.

Dazu wurden sämtliche Arrays mit der Affymetrix GeneChip DNA Analysis Software 2.0 (GDAS 2.0) analysiert, um Genotypbestimmungen für jeden SNP auf dem Array zu generieren. Die Genotypbestimmungen waren entweder eindeutige Bestimmungen wie homozygot AA, BB, heterozygot AB oder kein Signal. Die Kommentierung für jeden SNP wurde sowohl in GDAS 2.0 als auch das Webbasierte NetAffx^{™} Analysecenter gestellt.

Die Genotypdaten wurden dann in Microsoft Excel (Microsoft Corp., Washington, Vereinigte Staaten von Amerika) exportiert und zuerst nach der chromosomalen Lokalisierung eines jeden SNPs und anschließend nach dem Genotyp der Familienmitglieder sortiert. Eine UPD-Suche über das gesamte Genom erfolgte durch die Identifizierung von biallelischen Markern, die für ein Allel im Patienten homozygot und für das andere Allel in dem nicht übertragenden Elternteil homozygot vorlagen. Geclusterte SNPs, die entgegengesetzte Homozygotie zeigten, wurden als informative biallelische Marker identifiziert, die das Vorliegen einer UPD anzeigen und wurden kategorisiert als primäre informative Marker. Sämtliche anderen SNPs entlang eines Chromosoms oder in einem chromosomalen Segment, die flankiert waren von primären informativen Markern, wurden hinsichtlich der Differenzierung zwischen Iso- und Heterodisomie evaluiert. Diese SNPs wurden als sekundäre informative Marker klassifiziert. Nicht geclusterte Einzel-SNPs in irgendeiner chromosomalen Region, die keinerlei Mendel'sche Vererbung zeigen, wurden als falschinformative Marker betrachtet.

### Diagnoseergebnis

Die Ergebnisse der SNP-Genotypisierung unter Verwendung des 10K-Arrays sind in Tabelle 1 zusammengefasst.

**Tabelle 1: Zusammenfassung der Ergebnisse der Genotypisierung in Familien mit einem von uniparentaler Disomie (UPD) betroffenen Nachkommen**

| Familie | UPD | Individuum | SNP-Bestimmungsrate | Anzahl von Mi^{a} | Lokalisierung von Mi | Anzahl von Mf^{b} | Chromosomen für Mf^{c} |
|---|---|---|---|---|---|---|---|
| 1 | Paternal UPD15 | Patient; Angelm.-Syndrom | 85,06 % | 47 | 15q11.2-q26.3 | 9 | 1, 2, 5, 11(2), 12(3), 14 |
| | | Mutter | 88,83 % | | | | |
| 2 | Maternal UPD15 | Patient; Prader-Willi-Syndrom | 95,44 % | 33 | 15q12-q26.3 | 6 | 1(2), 4, 5, 6, 18 |
| | | Vater | 95,11 % | | | | |
| 3 | Maternal UPD7^{d} | Patient; Silver-Russel-Syndrom | 93,28 % | 84 | 7p22.2-q36.3 | 6 | 5, 8(2), 11, 14, 19 |
| | | Vater | 95,41 % | | | | |
| 4 | Paternal UPD2p | Patient; habituelle Aborte | 87,98 % | 65 | 2p25.3-p11.2 | 2 | 9, 16 |
| | Maternal UPD2q^{e} | Mutter | 85,14 % | 104 | 2q11.2-q37.2 | 4 | 4, 9, 11, 12 |
| | | Vater | 91,43 % | | | | |
| 5 | Paternal UPD11p^{↑} | Patient; Beckwith-Wiedemann-Syndrom | 85,62 % | 31 | 11p15.4-p11.2 | 16 | 2(3),4(2),5,6, 7,9(2),11(2), |
| | | Mutter | 92,13 % | | | | 14,16,18(2) |
| | | Vater | 91,84 % | | | | |
| 6 | Paternal UPD20q^{g} | Patient; Pseudo-Hypothyriodismus | 88,31 % | 13 | 20q12-q13.33 | 14 | 1,2,3,5(4),6, 7,8,9,10,11,12 |
| | | Mutter | 84,46 % | | | | |
| | | Vater | 92,74 % | | | | |

| | | | | | | | |
|---|---|---|---|---|---|---|---|
| ^{a}Mi = informative biallelische Marker, ^{b}Mf = falschinformative biallelische Marker, die keine Mendel'sche Vererbung zeigen, *^{c}* Falschinformative Marker waren auf hier aufgelisteten verschiedenen Chromosomen lokalisiert. Bei Feststellung von > 1 Mf auf irgendeinem einzelnen Chromosom ist die Anzahl dieser Mf in Klammern dargestellt, ^{d} Mergenthaler et al., 2000, Formation of uniparental disomy 7 delineated from new cases and a UPD7 case after trisomy 7 rescue. Presentation of own results and review of the literature. Ann Genet 43:15-21, ^{e} Albrecht et al., 2001; Uniparental isodisomy for paternal 2p and maternal 2q in a phenotypically normal female with two isochromosomes, i(2p) and i(2q). J Med Genet 38:214, ^{f} Borck et al., 2004, Genome-wide screening using automated fluorescent genotyping to detect cryptic cytogenetic abnormalities in children with idiopathic syndromic mental retardation. Clin Genet 66:122-127, ^{g} Bastepe et al., 2001, Paternal Uniparental Isodisomy of Chromosome 20q - and the Resulting Changes in GNAS1 Methylation - as a Plausible Cause of Pseudohypoparathyroidism. Am J Hum Genet 68:1283-1289. | | | | | | | |

Das durchgeführte Verfahren wäre dann für eine zuverlässige UPD-Diagnostik geeignet, wenn bei sämtlichen untersuchten Patienten die entsprechenden SNPs in homozygoter Ausprägung vorlägen und damit die nach dem Stand der Technik vorgenommene UPD-Diagnose bestätigt werden würde.

Die mittlere SNP-Bestimmungsrate in sämtlichen Familien betrug 90,2 % und bewegte sich von 84,46 % bis 95,44 %. Diese relativ geringeren Bestimmungsraten im Vergleich zum erwarteten Mittelwert von 95 % (Kennedy et al., 2003, Large-scale genotyping of complex DNA. Nat Biotechnol 21:1233-1237) ist möglicherweise auf das unterschiedliche Probenalter (alte Proben, wiederholendes Einfrieren und Auftauen) und die Variationen, die durch die Experimentatoren verursacht wurden (Middleton et al., 2004, Genomewide Linkage Analysis of Bipolar Disorder by Use of a High-Density Single-Nucleotide-Polymorphism (SNP) Genotyping Assay: A Comparison with Microsatellite Marker Assays and Finding of Significant Linkage to Chromosome 6q22. Am J Hum Genet 74: 886-897; Shrimpton et al., 2004, A HOX Gene Mutation in a Family with Isolated Congenital Vertical Talus and Charcot-Marie-Tooth Disease. Am J Hum Genet 74:886-897) zurückzuführen.

Um die Genauigkeit des Assays für das Detektieren eines UPDs abzuschätzen, wurden sämtliche Genotypenbestimmungen auf dem Array hinsichtlich Fehlerbestimmungsraten untersucht, die entgegengesetzte Homozygotie im Patienten und Elternteil (Nicht-Mendel'sche Vererbung) zeigten. Lediglich 0,03 bis 0,1 % der SNPs zeigten derartige falschpositive Bestimmungen, die in dieser Studie auch als falschpositive Marker definiert wurden (Tabelle 1). In isodisomischen chromosomalen Regionen in sämtlichen analysierten Patienten gab es lediglich drei falschnegative Bestimmungen (0,2 %). Diese SNPs waren jedoch auf verschiedenen Chromosomen lokalisiert und waren nicht in einer chrosomalen Region geclustert. Des Weiteren wurden die X-Chromosom-Genotypbestimmungen in acht männlichen Individuen analysiert. Dabei wurde für jedes SNP auf dem X-Chromosom eine Homozygotie auf Grund eines einzigen X-Chromosoms erwartet. Keines der SNPs, das auf dem X-Chromosom kartiert wurde, zeigte in den acht männlichen Individuen, die untersucht wurden, Heterozygotie (Daten nicht gezeigt).

Sämtliche der ausgewählten und über das gesamte Genom verteilten SNPs der untersuchten Patienten, die den informativen SNPs der Elternteile entsprachen, lagen in homozygotem Zustand vor und verifizieren damit die zuvor vorgenommene UPD-Diagnose.

In der Familie 2 (Maternal UPD 15) wurden 33 informative Marker auf dem Chromosom 15 identifiziert (Tabelle 1, Fig. 1). Die Genotypisierungsanalyse dieses Patienten ergab, dass heterozygote und homozygote Bestimmung randomisiert über das Chromosom verteilt waren, mit Ausnahme von zwei Clustern von homozygoten Bestimmungen bei 15 q14 bis q21.1 (Cluster 2) und bei 15 q26.2 bis qter (Cluster 4) (Fig. 2). Der Patient zeigte lediglich einen einzelnen heterozygoten SNP innerhalb des Clusters 2 (falschnegative Bestimmung). In nächster Nähe zu dem Centromer bei 15 q1.2 waren die ersten fünf SNPs in dem Patienten und dem Vater identisch homozygot, gefolgt von einem Cluster von heterozygoten SNPs (Cluster 1) (Fig. 2). Das Vorhandensein von heterozygoten Markern entlang des gesamten Chromosoms 15 korreliert mit einem Fehler in der Meiose.

Im Falle der paternalen UPD 15 (Familie 1) wurden 47 informative biallelische Marker auf dem Chromosom 15 detektiert, während im Falle der maternalen UPD 7 (Familie 3) 84 informative Marker auf dem Chromosom 7 identifiziert wurden (Tabelle 1, Fig. 1). In jedem Fall gab es lediglich eine heterozygote Bestimmung bei den 297 detektierten SNPs auf Chromosom 15 bzw. bei den 559 detektierten SNPs auf Chromosom 7 (falschnegative Bestimmungen). Diese Reduzierung auf Homozygotie über das gesamte Genom in beiden Fällen weist auf eine vollständige Isomie der entsprechenden Chromosomen hin, was auf einen postzygotischen Fehler zurückgeführt werden könnte.

Um die Leistung des erfindungsgemäßen Mikroarray-basierenden Verfahrens hinsichtlich der Identifizierung von segmentalen UPDs zu untersuchen, wurde der 10K-Array in drei verschiedenen Fällen untersucht, einer paternalen UPD 20q (Pseudo-Hypothyriodismus), einer paternalen UPD11p (Beckwith-Wiedemann-Syndrom) und einer paternalen UPD2p in Kombination mit einer maternalen UPD2q (habituelle Aborte). Der letztere Patient (Familie 4) wies ein seltenes chromosomales Rearrangement mit zwei Isochromosomen, i(2p) und i(2q) auf; vgl. Albrecht et al., 2001 (a.a.O.). Die beiden unterschiedlichen UPDs, die in diesem Patienten vorhanden waren, wurden separat evaluiert. Auf 2p und 2q konnten 65 bzw. 104 informative Marker detektiert werden (Tabelle 1, Fig. 1). Die Reduktion auf die Homozygotie von sämtlichen anderen SNPs auf 2p als auch auf 2q bestätigte paternale uniparentale Isodisomie 2p und maternale uniparentale Isodisomie 2q.

In Familie 5 (paternale UPD 11p) (Borck et al., 2004, a.a.O.), waren 31 informative Marker zwischen 11p15.4 und 11p11.2 lokalisiert (Fig. 1) mit Reduktion zur Homozygotie von sämtlichen biallelischen Markern, was eine Isodisomie in dieser Region anzeigt. Die SNPs in der Nähe der Centromere zeigten entweder eine normale biallelische Vererbung oder waren nichtinformativ.

In Familie 6 (paternale UPD20q) (Bastepe et al., 2001, a.a.O.) wurden über den langen Arm des Chromosoms 20 insgesamt 13 informative Marker identifiziert (Tabelle 1, Fig. 1). Sämtliche 91 detektierte SNPs einschließlich der informativen Marker zwischen 20q11.22 bis q13.33 zeigten eine Reduktion zur Homozygotie entsprechend einer Isodisomie dieses Segmentes. Erneut zeigten lediglich zwei SNPs in nächster Nähe zu dem Centromer (in 20q11.21) Heterozygotie in dem Patienten und den Eltern (Fig. 3). Die SNPs, die in 20p lokalisiert waren, zeigten normale Mendel'sche Vererbung (Fig. 3).

Parallel dazu wurden weitere Kontrollexperimente durchgeführt. Dabei wurden gesunde Probanden sowie deren dazugehörige Elternteile hinsichtlich informativer SNPs genotypisiert. Es zeigte sich, dass die entsprechenden SNPs der gesunden Probanden ausschließlich in heterozygotem Zustand vorlagen.

Diese Ergebnisse belegen, dass bei dem Vorliegen einer homozygoten Ausprägung der SNPs beim untersuchten Patienten, die den informativen SNPs der Eltern entsprechen, eine Korrelation mit dem Vorliegen einer UPD gegeben ist. Mit dem erfindungsgemäßen Verfahren gelingt deshalb eine zuverlässige Diagnose für uniparentale Disomie basierend auf der genomweiten Analyse der Vererbung von informativen SNPs.

## Patentansprüche

1. Verfahren zur Diagnose von uniparentaler Disomie (UPD) bei einem menschlichen Lebewesen, das folgende Schritte aufweist:
(1) Bereitstellung einer biologischen Probe des menschlichen Lebewesens sowie von biologischen Proben beider Elternteile des menschlichen Lebewesens;
(2) Genotypisierung von Singel-Nukleotid-Polymorphismen (SNPs) des Lebewesens und der beiden Elternteile anhand der biologischen Proben;
(3) Auswahl von solchen SNPs, die bei den beiden Elternteilen diametral entgegengesetzt homozygot vorliegen (informative SNPs);
(4) Vergleich der Genotypen der informativen SNPs mit dem Genotyp der entsprechenden SNPs des menschlichen Lebewesens, und
(5) Korrelation einer Homozygotie der SNPs des menschlichen Lebewesens aus Schritt (4) mit dem Vorliegen einer UPD.

2. Verfahren nach Anspruch 1, **dadurch gekennzeichnet, dass** in Schritt (2) die Genotypisierung von SNPs von einem Chromosom erfolgt.

3. Verfahren nach Anspruch 1, **dadurch gekennzeichnet, dass** in Schritt (2) eine genomweite Genotypisierung von SNPs erfolgt.

4. Verfahren nach einem der Ansprüche 1 bis 3, **dadurch gekennzeichnet, dass** das menschliche Lebewesen Anzeichen einer intrauterinen Wachstumsretardierung (IUGR) mit einer postnatalen Entwicklungsverzögerung (PGR) zeigt.

5. Verfahren nach einem der Ansprüche 1 bis 3, **dadurch gekennzeichnet, dass** das menschliche Lebewesen Anzeichen eines Syndroms zeigt, das ausgewählt ist aus der Gruppe bestehend aus: Prader-Willi-Syndrom (PWS), Angelmann-Syndrom (AS), Silver-Russell-Syndrom (SRS) und Beckwith-Wiedemann-Syndrom.

6. Verfahren nach einem der Ansprüche 1 bis 3, **dadurch gekennzeichnet, dass** das menschliche Lebewesen Anzeichen transienter neonataler Diabetes mellitus zeigt.

7. Verwendung von in einer biologischen Probe eines menschlichen Lebewesens vorliegenden SNPs, die bei beiden Elternteilen diametral entgegengesetzt homozygot vorliegen (informative SNPs), zur Diagnose von UPD, insbesondere im Zusammenhang mit Verdacht auf IUGR/PGR, PWS, AS, SRS, Beckwith-Wiedemann-Syndrom oder transienter neonataler Diabetes mellitus.

## Claims

1. A method for diagnosing uniparental disomy (UPD) in a human being which comprises the following steps:
(1) provision of a biological sample of the human being and of biological samples of both parents of the human being;
(2) genotyping of single nucleotide polymorphisms (SNPs) of the human being and of both parents on the basis of the biological samples;
(3) selection of such SNPs, which in both parents are present in a diametrical opposite homozygous form (informative SNPs);
(4) comparison of the genotypes of the informative SNPs with the genotype of the corresponding SNPs of the human being, and
(5) correlation of a homozygosity of the SNPs of the human being from step (4).

2. The method of claim 1, **characterized in that** in step (2) the genotyping of SNPs of one chromosome is performed.

3. The method of claim 1, **characterized in that** in step (2) a genome-wide genotyping of SNPs is performed.

4. The method of any of claims 1 to 3, **characterized in that** the human being shows signs of an intrauterine growth retardation (IUGR) with a postnatal developmental retardation (PGR).

5. The method of any of claims 1 to 3, **characterized in that** the human being shows signs of a syndrome which is selected from the group consisting of: Prader-Willi syndrome (PWS), Angelmann syndrome (AS), Silver-Russell syndrome (SRS) and Beckwith-Wiedemann syndrome.

6. The method of any of claims 1 to 3, wherein the human being shows signs of transient neonatal diabetes mellitus.

7. Use of SNPs present in a biological sample, which in both parents are present in diametrical opposite homozygous form (informative SNPs) for diagnosing UDP, in particular in connection with a suspicion on IUGR/PGR, PWS AS, SRS, Beckwith-Wiedemann syndrome or transient neonatal diabetes mellitus.

## Revendications

1. Procédé de diagnostic de la disomie uniparentale (UPD) chez un être humain, présentant les étapes suivantes :
(1) Mise à disposition d'un échantillon biologique de l'être humain et d'échantillons biologiques des deux parents de l'être humain ;
(2) génotypage des polymorphismes d'un nucléotide simple (SNP) de l'être humain et des deux parents sur la base des échantillons biologiques ;
(3) Sélection de ces SNP qui sont homozygotes diamétralement opposés (SNP informatifs) chez les deux parents ;
(4) Comparaison des génotypes des SNP informatifs avec le génotype des SNP correspondants de l'être humain, et
(5) Corrélation d'une homozygotie des SNP de l'être humain de l'étape (4) avec la présence d'une UPD.

2. Procédé selon la revendication 1, **caractérisé en ce qu'**à l'étape (2) s'effectue le génotypage des SNP d'un chromosome.

3. Procédé selon la revendication 1, **caractérisé en ce qu'**un génotypage des SNP est effectué sur tout le génome à l'étape (2).

4. Procédé selon l'une des revendications 1 à 3, **caractérisé en ce que** l'être humain présente des signes d'un retard de croissance intra-utérin (IUGR) avec un retard de développement post-natal (PGR).

5. Procédé selon l'une des revendications 1 à 3, **caractérisé en ce que** l'être humain présente des signes d'un syndrome choisi dans le groupe comprenant : le syndrome de Prader-Willi (PWS), le syndrome d'Angelmann (AS), le syndrome de Silver-Russel (SRS) et le syndrome de Beckwith-Wiedermann.

6. Procédé selon l'une des revendications 1 à 3, **caractérisé en ce que** l'être humain présente des signes de diabète sucré néonatal provisoire.

7. Utilisation de SNP présents dans un échantillon biologique d'un être humain qui sont homozygotes diamétralement opposés chez les deux parents (SNP informatifs) pour le diagnostic de l'UPD, en particulier en rapport avec une suspicion de IUGR/PGR, de syndrome de PWS, AS, SRS, Beckwith-Wiedermann ou de diabète sucré néonatal provisoire.
